# EUROPÄISCHE PATENTSCHRIFT

(11) **EP 2 786 726 B1**
(45) Veröffentlichungstag und Bekanntmachung des Hinweises auf die Patenterteilung: **07.06.2023**
(21) Anmeldenummer: 14164683.6
(22) Anmeldetag: 12.01.2006
(51) Int. Cl.: A61F 2/24, A61F 2/95

(54) **Katheter für die transvaskuläre Implantation von Herzklappenprothesen**
Catheter for transvascular implantation of heart valve prosthetics
Cathéter pour l'implantation trans-vasculaire de prothèses de valvules cardiaques

(30) Priorität: 20.01.2005 DE 102005003632
(43) Veröffentlichungstag der Anmeldung: 08.10.2014
(62) Teilanmeldung aus: 11162971.3
(73) Patentinhaber: JenaValve Technology, Inc., Irvine, CA 92618 (US)
(72) Erfinder: Ottma, Rüdiger, 99441 Grossschwabhausen (DE); Moszner, Robert, 07639 Bad-Klosterlausnitz (DE); Damm, Christoph, 07743 Jena (DE); Figulla, Hans-Reiner, 07749 Jena (DE); Ferrari, Markus, 07747 Jena (DE)
(74) Vertreter: Meissner Bolte Partnerschaft mbB

(56) Entgegenhaltungen:
- WO-A1-03/022183
- WO-A1-2004/019825
- M FERRARI: "Transarterial aortic valve replacement with a self expanding stent in pigs", HEART, Bd. 90, Nr. 11, 1. November 2004 (2004-11-01), Seiten 1326-1331, XP055137208, ISSN: 1355-6037, DOI: 10.1136/hrt.2003.028951

## Beschreibung

Die Erfindung betrifft Katheter für die transvaskuläre Implantation von Herzklappenprothesen mit selbstexpandierenden Verankerungsstützen, mit denen eine minimal-invasive Implantation von Herzklappenprothesen möglich ist.

Häufig und mit steigender Anzahl müssen an Patienten Herzklappenprothesen implantiert werden, wobei sowohl künstliche, wie auch biologische Implantate für Herzklappenprothesen eingesetzt werden.

Bisher werden solche Operationen so durchgeführt, dass am narkotisierten Patienten eine Herz-Lungen-Maschine eingesetzt werden muss. Dabei handelt es sich um einen kostenintensiven operativen Eingriff, bei dem die jeweiligen Patienten einer hohen psychisehen und physischen Belastung ausgesetzt sind. Das Letalitätsrisiko sollte unter 3 % gehalten sein. Mit ansteigendem Alter der jeweiligen Patienten sowie auch durch fortgeschrittene Beeinträchtigung der jeweiligen Herzklappen kommt es dazu, dass Patienten, die eigentlich behandlungsbedürftig sind, inoperabel sind. Da bei diesen Patienten ein operativer Klappenersatz nicht möglich ist, leiden sie unter verringerter Lebensqualität und weisen eine erheblich reduzierte Lebenserwartung auf. Das Risiko des Eingriffs wäre extrem hoch.

Dieser Sachverhalt trifft auch auf Operationen zu, bei denen Herzklappenprothesen mit Verankerungssystemen mittels so genannter Ballon-Katheter implantiert werden.

Bei einer solchen Vorgehensweise können Fehlpositionierungen auftreten, die für den Patienten erhebliche Folgen aufweisen können, was bis zum Tode des jeweiligen Patienten führen kann.

So wurde in der jüngeren Vergangenheit versucht, Herzklappenprothesen mittels minimal-invasiver Eingriffe zu implantieren, wobei solche Prothesen mit Verankerungsstützen über die Aorta eines Patienten ein und durch die Aorta bis zum Herzen geführt werden. Bei Erreichung des Implantationsortes am Herzen wurde eine Selbstexpansion solcher Verankerungsstützen, an denen eine Herzklappenprothese befestigt ist, initiiert, die zu einer sicheren Fixierung und exakten Positionierung der Herzklappenprothese führen sollte. Solche Verankerungsstützen wurden aus Formgedächtnislegierungen, wie beispielsweise "Nitinol" vorab hergestellt und dabei wurde die Legierung so ausgewählt, dass ihre Sprungtemperatur bei ca. 37 °C liegt und nach Erreichen der Sprungtemperatur die Selbstexpansion auslösbar ist.

Durch eine solche Expansion spannt sich die Verankerungerungsstütze so auf, dass sie sich an der Aortenwandung anlegen kann und gegebenenfalls mittels zusätzlicher Widerhakenelemente dort sicher fixiert werden kann. Gleichzeitig wird die Herzklappenprothese aufgefaltet, so dass sie ihre Funktion übernehmen kann.

Eine solche Verankerungsstütze mit Herzklappenprothese ist beispielsweise in WO 2004/019825 A1 beschrieben.

An einer solchen Verankerungsstütze sind proximalseitig Stützbügel ausgebildet, die in Taschen der Herzklappe eines Patienten eingeführt werden können, so dass die Verankerungsstütze bei einem chirurgischen Eingriff mittels dieser Stützbügel sehr genau positioniert werden kann. An dieser Verankerungsstütze sollen zusätzlich so genannte Kommissurenbügel ausgebildet sein, die gemeinsam mit den Stützbügeln Teile der alten Herzklappe eines Patienten nach erfolgter Entfaltung der Verankerungsstütze einklemmen, so dass infolge dieser Klemmwirkung die Verankerungsstütze sicher positioniert und fixiert werden kann.

Die Stütz- und Kommissurenbügel dieser bekannten Verankerungsstütze sollen dabei so angeordnet und auch dimensioniert sein, dass eine sequentielle Selbstexpansion erfolgen kann. Dies bedeutet, dass die Verankerungsstütze bei der Implantation innerhalb einer Kartusche aufgenommen ist. Sie wird dann über einen Katheter durch Aorta bis hin zum erkrankten Herzen geführt. Nach Erreichung des Implantationsortes soll die Kartusche so manipuliert werden, dass eine Freigäbe der Stützbügel für ihre Selbstexpansion erfolgen kann. Nachfolgend wird die Kartusche mit Verankerungsstütze so bewegt und ausgerichtet, dass die Stützbügel in die Taschen der Herklappen des jeweiligen Patienten eingeführt werden. Dadurch kann eine exakte Positionierung erreicht werden.

Im Anschluss daran wird die jeweilige Kartusche wiederum weiter manipuliert, so dass auch die Kommissurenbügel freigegeben werden und selbst expandieren können. Dabei wird die alte Herzklappe zwischen Stütz- und Kommissurenbügel eingeklemmt und die Herzklappenprothese in ihre entfaltete Funktionsstellung aufgespannt.

Nach erfolgter Implantation von Verankerungsstütze mit Herzklappenprothese kann dann der Katheter mit der Kartusche wieder über die Aorta aus dem Körper des Patienten entfernt werden.

Obwohl, wie bereits angesprochen, mit Hilfe der an der Verankerungsstütze ausgebildeten Stützbügel eine deutlich vereinfachte und verbesserte Positionierung der zu implantierenden Herzklappenprothese erreicht werden kann, besteht die Möglichkeit, dass es zu Fehlimplantationen kommen kann und die Herzklappenprothese nicht oder nur unbefriedigend funktionsfähig ist. Eine Entfernung einer so nicht oder unbefriedigenden funktionsfähigen Herzklappenprothese ist dann in einigen Fällen nicht mehr möglich bzw. es besteht für den jeweiligen Patienten ein erhöhtes Mortalitätsrisiko.

Ein weiteres Problem bei solchen chirurgischen Eingriffen stellt dabei auch der Aortenbogen am menschlichen Körper dar, der beim Einführen über die Aorta durchdrungen werden muss. Dabei muss bei der Bewegung von Kartusche und dem jeweiligen Katheter eine Richtungsänderung von ca. 180° mit einem relativ geringen Radius von ca. 50 mm durchgeführt werden, ohne dass es zu einer Verletzung der Gefäßwand kommt.

Es ist daher Aufgabe der Erfindung, das Patientenrisiko bei einer Implantation von Herzklappenprothesen zu reduzieren.

Erfindungsgemäß wird diese Aufgabe mit einem Katheter, das die Merkmale des Anspruchs 1 aufweist, gelöst. Vorteilhafte Ausgestaltungsformen und Weiterbildungen der Erfindung können mit den in den untergeordneten Ansprüchen bezeichneten Merkmalen erreicht werden.

In Verbindung mit dem erfindungsgemäßen Katheter kann in bevorzugter Form eine Herzklappenprothese mit selbstexpandierender Verankerungsstütze, wie sie aus WO 2004/019825 A1 bekannt ist und auf deren Offenbarungsgehalt hiermit Bezug genommen wird, eingesetzt werden.

Dabei sind Verankerungsstütze mit der daran befestigten Herzklappenprothese während der Implantation innerhalb einer Kartuscheneinheit temporär in zusammengefalteter Form aufgenommen.

Eine so vorbereitete Kartuscheneinheit ist an einem Führungssystem proximalseitig lösbar befestigt. Kartuscheneinheit und Führungssystem sind mit ihrem Aussendurchmesser insoweit minimiert, dass sie ohne weiteres durch eine Aorta eines zu operierenden Patienten hindurchgeführt werden können, wobei der gesamte innerhalb der Aorta zur Verfügung stehende freie Querschnitt nicht vollständig ausgefüllt sein sollte.

Das Führungssystem hat dabei eine ausreichend große Länge, um die Kartuscheneinheit mit dem Führungssystem über eine Einführung an der Leiste eines Patienten durch die Aorta bis hin zum Herzen des Patienten führen zu können.

Am Führungssystem ist ein flexibler, biegbarer Bereich vorhanden, mit dem ein Biegeradius und Biegewinkel realisiert werden kann, der dem Aortenbogen eines Patienten folgen und berücksichtigen kann.

Durch das im Inneren hohle Führungssystem sind Betätigungselemente zur Kartuscheneinheit geführt. Mit diesem Betätigungselement können Teile der Kartuscheneinheit in gezielter Form manipuliert und bewegt werden. So kann mit den Betätigungselementen eine radiale oder auch laterale Bewegung von Teilen der Kartuscheneinheit erreicht werden. Mit Hilfe dieser gezielten Bewegung von Teilen der Kartuscheneinheit kann dann eine sequentielle Freigabe von Teilen der Verankerungsstütze erreicht werden, so dass die Implantation mit Verankerung, wie im einleitenden Teil der Beschreibung und in WO 2004/019825 A1 beschrieben, erfolgen kann.

So wird eine Freigabe von Stützbügeln einer Verankerungsstütze durch eine Verdrehung oder eine laterale Bewegung in proximaler oder distaler Richtung eines Teiles der Kartuscheneinheit erreicht und es werden dabei aber andere Teile, wie beispielsweise Kommissurenbügel noch innerhalb der Kartuscheneinheit in zusammengefalteter Form gehalten, die dann nachfolgend durch eine entsprechende Bewegung eines weiteren Teiles der Kartuscheneinheit oder einer weiterführenden Bewegung des gleichen Teiles der Kartuscheneinheit das vorab die Stützbügel noch im zusammengefalteter Form innerhalb der Kartuscheneinheit gehalten hat, für eine Expansion freigegeben werden können.

Das Aufspannen der Herzklappenprothese, die an der Verankerungsstütze, beispielsweise durch Vernähen befestigt worden ist, wird dann gleichzeitig mit der Expansion der jeweiligen Bügel der Verankerungsstütze, an denen die Herzklappenprothese befestigt ist, erreicht.

In bevorzugter Ausführungsform sind neben den Betätigungselementen für Teile der Kartuscheneinheit weitere Betätigungselemente durch das innen hohle Führungssystem geführt, die am biegbaren Bereich für eine gezielte Beeinflussung seiner Krümmung angreifen.

Insbesondere durch die Auslösung von Zugkräften über die Betätigungselemente kann eine gezielte Krümmung des biegbaren Bereiches während der Implantation beim Durchdringen des Aortenbogens erreicht werden. So können als Betätigungselemente Zugseile oder Zugdrähte durch das innen hohle Führungssystem bis an den proximalen Rand des biegbaren Bereiches geführt und dort am Führungssystem befestigt sein, wobei die Kraftangriffspunkte von zwei solcher Betätigungselemente dann sich diametral gegenüberliegend angeordnet sein sollten und außerdem eine Anordnung, um jeweils 90° zur Biegeachse, um die der biegbare Bereich gekrümmt werden soll, gewählt werden.

So kann eine gezielte Beeinflussung der Krümmung des biegbaren Bereiches durch Ausübung einer Zugkraft über eines der vorhandenen Betätigungselemente erreicht werden, wenn das Führungssystem mit dem biegbaren Bereich durch den Aortenbogen geschoben bzw. nach erfolgter Implantation aus diesem herausgezogen wird.

Der biegbare Bereich eines Führungssystems kann dabei in Form einer Gliederkette ausgebildet sein, in dem die einzelnen Glieder über Einzelgelenke miteinander verbunden sind. Die Einzelgelenke greifen dabei formschlüssig in jeweils benachbarte Glieder ein. Sie sind dabei so ausgebildet, dass eine Krümmung des biegbaren Bereichs um mehr als 180° mit einem Biegeradius, der gewährleistet, dass mindestens der Radius des Aortenbogens erreichbar ist, eingehalten werden können.

Die Einzelgelenke an den einzelnen Gliedern einer Gliederkette sollten dabei ebenfalls sich paarweise diametral gegenüberliegend an den einzelnen Gliedern ausgebildet und parallel zur Drehachse des biegbaren Bereiches angeordnet sein.

Das an einem erfindungsgemäßen Katheter eingesetzte Führungssystem sollte vorteilhaft auch so ausgebildet sein, dass ein flüssiges Kühlmittel bzw. Pharmaka durch das innen hohle Führungssystem bis zur Kartuscheneinheit geführt werden kann. Mit Hilfe eines solchen flüssigen Kühlmittels, beispielsweise einer Kochsalzlösung kann die Verankerungsstütze unterhalb der Sprungtemperatur, der Formgedächtnislegierung gehalten werden. Außerdem kann dadurch auch vermieden werden, dass Körperflüssigkeit in das Innere des Führungssystems eintreten kann, wobei ein entsprechender Flüssigkeitsdruck eingehalten werden sollte, der dem Eindringen von Körperflüssigkeit oder in Körperflüssigkeit enthaltenden weiteren Bestandteilen einen ausreichend großen Widerstand leistet.

Mit entsprechender Einführung des flüssigen Kühlmittels kann aber auch verhindert werden, dass Gas, beispielsweise Luft, in die Aorta und das Blut gelangen kann.

Hierzu sollte das gesamte Führungssystem möglichst flüssigkeitsdicht ausgebildet sein. Dementsprechend kann ein flexibler, biegbarer Bereich, der in Form einer Gliederkette ausgebildet ist, mit Hilfe eines Kunststoffschlauches von außen flüssigkeitsdicht abgedichtet sein.

Die Teile der Kartuscheneinheit, die für eine Freigabe von Bügeln der Verankerungsstütze gezielt bewegt werden können, sind bevorzugt als hülsenförmige Elemente ausgebildet, deren Innen- und Außendurchmesser so aufeinander abgestimmt sind, dass sie teleskopförmig ineinander greifen können, wobei mindestens zwei der hülsenförmigen Elemente so aufeinander abgestimmte Innen- und Außendurchmesser aufweisen, dass zwischen ihnen eine zusammengefaltete Verankerungsstütze mit Herzklappenprothese aufgenommen ist und in zusammengefalteter Form gehalten werden kann.

Beim Einführen des Katheters sollte die Kartuscheneinheit möglichst vollständig geschlossen sein und für ein erleichtertes Einführen durch die Aorta proximalseitig eine Spitze aufweisen, die wiederum besonders bevorzugt aus einem flexiblen Werkstoff, beispielsweise Silikon gebildet ist.

Bei Erreichen der Kartuscheneinheit des Herzens des jeweiligen Patienten kann dann die entsprechende Manipulation, also die Bewegung von Teilen/hülsenförmigen Elementen der Kartuscheneinheit so durchgeführt werden, dass die unterschiedlichen Bügel der Verankerungsstütze sequentiell freigegeben werden und gleichzeitig die daran befestigte Herzklappenprothese aufgespannt wird.

Dabei bleibt aber die Verankerungsstütze noch an der Kartuscheneinheit fixiert gehalten. Hierfür sind an einem hülsenförmigen Element der Kartuscheneinheit Verankerungselemente ausgebildet, die distal, beispielsweise an dort an der Verankerungsstütze ausgebildeten Ösen angreifen. Diese Verankerungselemente sind in dieser Position gemeinsam mit dem distalen Teil der Verankerungsstütze von einem hülsenförmigen Element der Kartuscheneinheit auch überdeckt, so dass der distale Teil der Verankerungsstütze noch zusammengefaltet gehalten wird.

In dieser Stellung ist eine Überprüfung der Funktion der bereits entfalteten Herzklappenprothese möglich. Nach erfolgtem Funktionsnachweis der Herzklappenprothese kann dann eine weitere Manipulation, durch entsprechende Bewegung des vorab die Verankerungselemente mit distalem Teil der Verankerungsstütze überdeckenden hülsenförmigen Elementes, erfolgen, die zu einer vollständigen Freigabe auch des distalen Teiles der Verankerungsstütze und dementsprechend zur vollständigen Entfaltung führt.

Wird bei der Prüfung festgestellt, dass die implantierte Herzklappenprothese ihre Funktion nicht oder nur unzulänglich erfüllen kann, besteht in besonders vorteilhafter Weise die Möglichkeit durch entsprechend entgegengesetzt gerichtete Bewegung der Teile/hülsenförmigen Elemente die Verankerungsstütze mit Herzklappenprothese wieder in die Kartuscheneinheit zurückzubewegen und sämtliche Teile, also das gesamte Katheter durch die Aorta aus dem Körper des Patienten wieder zu entfernen, so dass das Operationsrisiko erheblich reduziert und nachfolgend ein weiterer Versuch einer Implantation am selben Patienten durchgeführt werden kann.

In einer vorteilhaften Ausführungsform des erfindungsgemäßen Katheters kann auch ein Führungsdraht durch den gesamten Katheter hindurchgeführt sein. Solche Führungsdrähte werden bei ähnlichen Operationen bereits eingesetzt, wobei sie vor dem Einführen des Katheters durch die Aorta des Patienten bis hinter das Herz geführt werden. Der Katheter kann dann über Kartuscheneinheit und Führungssystem in den Führungsdraht eingefädelt und entlang dessen in die Aorta bis hin zum Herzen des Patienten eingeschoben werden.

Für eine Überwachung des Einführvorganges des Katheters und auch die Manipulation des biegbaren Bereiches, insbesondere im Aortenbogen ist es vorteilhaft am Führungssystem und/oder der Kartuscheneinheit Markierungselemente vorzusehen, die aus einem Werkstoff gebildet sind, der Röntgenstrahlung absorbiert, so dass sich während der Operation die jeweilige Position an einem Röntgenbild erkennen lässt.

An einem erfindungsgemäßen Katheter kann aber auch ein Schirmfilter eingesetzt werden, mit dem das Eindringen von Partikeln in die Blutbahn des jeweiligen Patienten verhindert werden kann. Ein solches Schirmfilter kann am Führungssystem so befestigt sein, dass es dieses radial vollständig umgreift. Dabei sollte es elastisch vorgespannt sein, so dass es sich an die Gefäßwand an die Aorta anlegt und ein partikeldichter Verschluss gewährleistet werden kann.

Der erfindungsgemäße Katheter kann außerdem zusätzlich mit einem herkömmlichen Ballon ausgebildet sein, der im Inneren des Führungssystems oder der Kartuscheneinheit angeordnet und dort mitgeführt bzw. auch durch das Innere des Führungssystems bis hin zur expandierenden Verankerungsstütze geführt werden kann. Mit einem solchen Ballon, der beispielsweise durch eine unter erhöhtem Druck stehende Flüssigkeit in seinem Volumen entsprechend vergrößert werden kann, kann die Expansion der Verankerungsstütze noch unterstützt werden.

Die bereits angesprochenen, durch das Innere des Führungssystems geführten Betätigungselemente, die als Zug- und Druckmittel ausgebildet sein können, werden von einem Handhabungsteil aus manipuliert. Das Handhabungsteil kann als Handgriff ausgebildet sein, über den dann die Bewegung für das Einführen des Katheters vom jeweiligen Operateur ausgeübt werden kann.

An einem solchen Handhabungsteil sind dann weitere Bedienelemente vorhanden, über die die jeweilige Bewegung der Betätigungselemente ausgelöst werden kann. Dabei erfolgt die entsprechende Bewegung in dosierter Form, beispielsweise mit entsprechenden Übersetzungsverhältnissen, und eine Begrenzung der jeweiligen Bewegung wird durch Endanschläge oder Rasteinstellungen bewirkt. Dadurch lassen sich bestimmte maximale Wege oder Winkel einhalten, die für die sequentielle Expansion der Verankerungsstütze oder die gezielte Beeinflussung der Krümmung des biegbaren Bereiches berücksichtigt werden können. Dabei sollte eine möglichst feine Justierung der Endanschläge bzw. einzelner Raster möglich sein.

Sämtliche Teile eines erfindungsgemäßen Katheters, zumindest jedoch die die mit dem jeweiligen Patienten in unmittelbarem Kontakt treten bzw. auch in die Aorta eingeführt werden, sollten aus biokompatiblen Werkstoffen hergestellt worden sein, die vom jeweiligen Organismus vertragen werden können. Zusätzlich sollte eine Sterilisation möglich sein, wobei zumindest eines der gängigen Sterilisationsverfahren einsetzbar sein sollte.

Nachfolgend soll die Erfindung beispielhaft näher erläutert werden.

Dabei zeigen:
- Figuren 1 bis 4: ein Beispiel eines erfindungsgemäßen Katheters bei unterschiedlichen möglichen Phasen eines Implantationsvorganges in schematischer Form;
- Figur 5: ein Beispiel eines Katheters mit einem Handhabungsteil und
- Figur 6: das Handhabungsteil nach Figur 5 in einer Explosionsdarstellung.

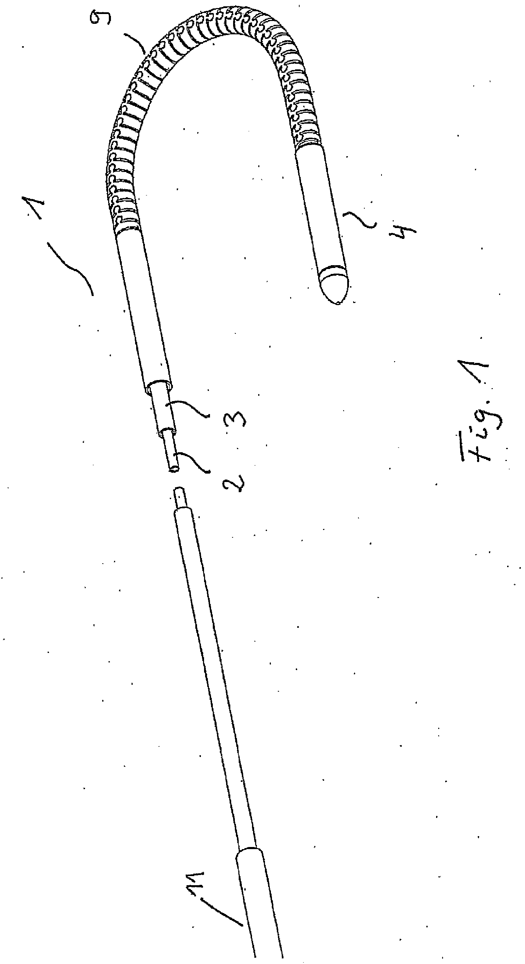

Mit den Figuren 1 bis 4 soll ein Beispiel für einen erfindungsgemäßen Katheter dargestellt und besser verständlich gemacht werden. Dabei sind in den einzelnen Abbildungen unterschiedliche Phasen, die während einer Implantation einer Verankerungsstütze 10 mit Herzklappenprothese durchlaufen werden können, dargestellt.

In Figur 1 ist das Beispiel eines erfindungsgemäßen Katheters, so gezeigt, dass in der Kartuscheneinheit 4, die noch vollständig geschlossen ist, eine Verankerungsstütze 10 mit Herzklappenprothese in nicht expandierter Form und entsprechend zusammengefaltet aufgenommen ist und so mittels des inneren hohlen Führungssystems 1 über einen geeigneten Zugang in die Aorta und durch diese zum jeweiligen Implantationsort am Herzen des Patienten hindurchgeführt werden kann.

An der Kartuscheneinheit 4 ist proximal eine flexible Spitze aus Silikon vorhanden, mit der das Einführen erleichtert und Verletzungsgefahren reduziert werden können.

Das Teil 5 der Kartuscheneinheit ist lösbar mit den anderen Teilen des Führungssystems 1, beispielsweise durch eine Verschraubung, mit diesem verbunden.

An die Kartuscheneinheit 4 schließt sich ein biegbarer Bereich 9 an, der so ausgebildet und dimensioniert ist, dass eine problemlose Bewegung durch den Aortenbogen eines Patienten gewährleistet werden kann.

Auf Möglichkeiten zur Ausbildung eines solchen biegbaren Bereiches 9 soll später noch zurückgekommen werden.

Im Übrigen sind weitere Teile des innen hohlen Führungssystems 1 dargestellt, wobei in den Figuren 1 bis 4 zwei Betätigungselemente 2 und 3 durch das Führungssystem 1 bis zur Kartuscheneinheit 4 geführt sind, dabei ist das Betätigungselement 2, hier durch das ebenfalls innen hohle Betätigungselement 3 hindurch, bis zur Kartuscheneinheit 4 geführt.

Die Betätigungselemente 2 und 3 sind hier als Druckfederstränge, die bevorzugt mittels Zugdrähten verstärkt sind, ausgeführt. Durch solche Zugdrähte kann die Sicherheit bei der Entfernung des Katheters aus dem Körper des Patienten nach erfolgter Operation erhöht werden.

Links sind weitere Teile 11 des Führungssystems 1 dargestellt, die beispielsweise als mehr oder weniger flexible hülsenförmige Teile ausgebildet sein können, wobei jedoch gesichert sein soll, dass sie für das Einführen in die Aorta und das wieder Herausführen aus der Aorta eine ausreichend hohe Druck- und Zugfestigkeit aufweisen. Hier können entsprechend steife Kunststoffschläuche, beispielsweise PTFE-Schläuche oder PTFE-basierte Schläuche eingesetzt werden, da sie für den Organismus ausreichend verträglich und auch sterilisierbar sind.

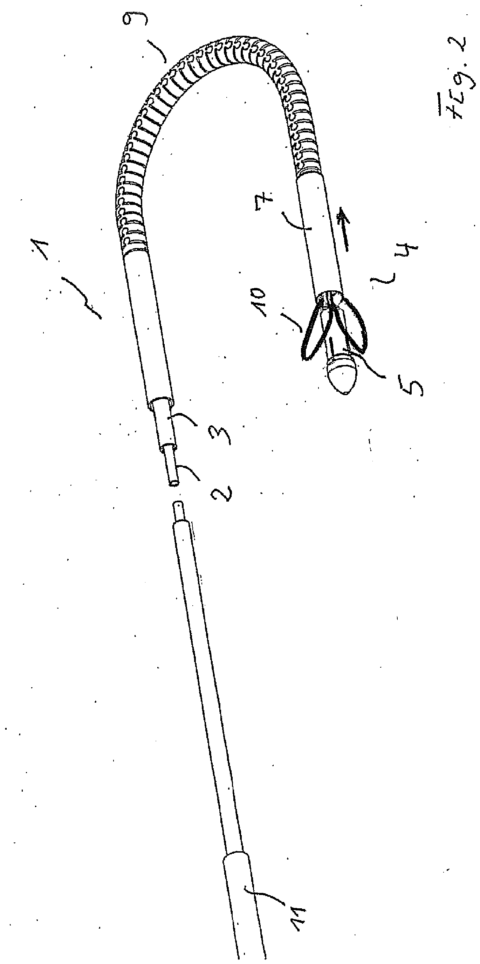

In Figur 2 ist dargestellt, wie in einem ersten Operationsstadium, nach Erreichen des Implantationsortes am Herzen des jeweiligen Patienten verfahren werden kann. Durch eine distale Bewegung eines der Betätigungselemente 2 und/oder 3 kann das Teil/hülsenförmige Element 7 der Kartuscheneinheit 4 distal zurückgezogen werden, so dass einige Bügel der Verankerungsstütze 10, beispielsweise und bevorzugt die Stützbügel, wie sie bei der aus WO 2004/019825 A1 bekannten Herzklappenprothese vorhanden sein sollen, expandieren und radial nach außen aufspannen.

So kann das gesamte Katheter mit Führungssystem 1 und der Kartuscheneinheit 4 proximal verschoben und so diese Bügel (Stützbügel) in die Taschen der alten Herzklappe des Patienten eingeführt werden. Verspürt der Operateur dann einen merklichen Widerstand, dürfte der Einführvorgang der Stützbügel, der Verankerungsstütze 10 in die Taschen der alten Herzklappe erfolgt sein.

Das Teil/hülsenförmige Element 5 der Kartuscheneinheit 4 kann dann weiter distal nach vorn bewegt werden, so dass auch weitere Bügel der Verankerungsstütze für eine Selbstexpansion freigegeben werden und die Herzklappenprothese aufgespannt wird.

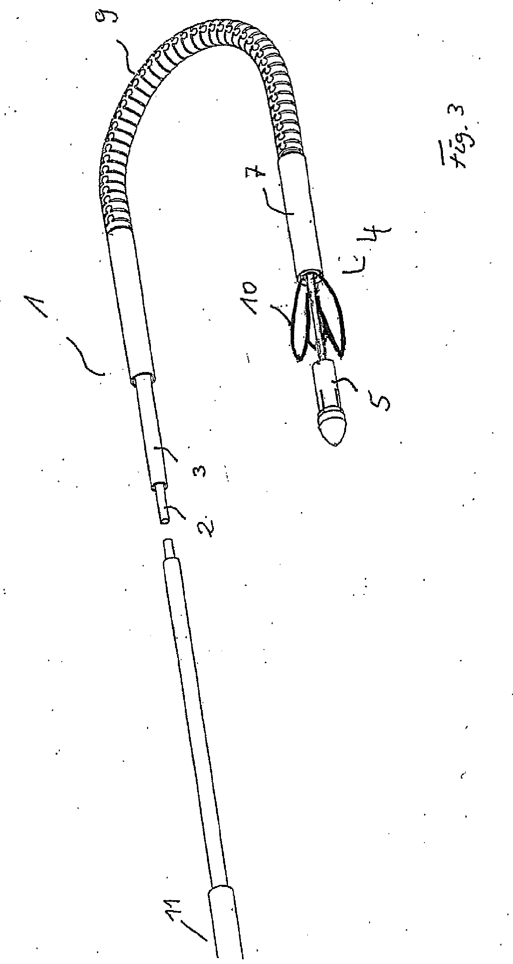

Eine Vorstufe hiervon ist in Figur 3 dargestellt, wobei hier noch keine vollständige Entfaltung einer Herzklappenprothese erreicht worden ist und auch noch keine vollständige Verankerung der Verankerungsstütze 10 ermöglicht wurde.

Mit Figur 3 wird außerdem deutlich, dass ein distaler Teil der Verankerungsstütze 10 noch innerhalb der Kartuscheneinheit 4, unterhalb des Teiles/hülsenförmigen Elementes 7 in der Kartuscheneinheit 4 aufgenommen ist. Dies bleibt solange der Fall, bis die Entfaltung und Positionierung der Herzklappenprothese in soweit erfolgt ist, dass diese auf ihre Funktionalität überprüft werden konnte.

Wird bei einer solchen Überprüfung eine Fehlfunktion oder Fehlpositionierung erkannt, kann das Teil/hülsenförmige Element 7 wieder über eines der beiden Betätigungselemente 2 oder 3 proximal verschoben werden, so dass die Verankerungsstütze 10 mit Herzklappenprothese zumindest teilweise wieder in die Kartuscheneinheit 4 aufgenommen wird und dann das gesamte Katheter aus dem Patienten durch Herausziehen aus der Aorta entfernt werden kann, ohne dass es zu Verletzungen der Gefäßwand kommt.

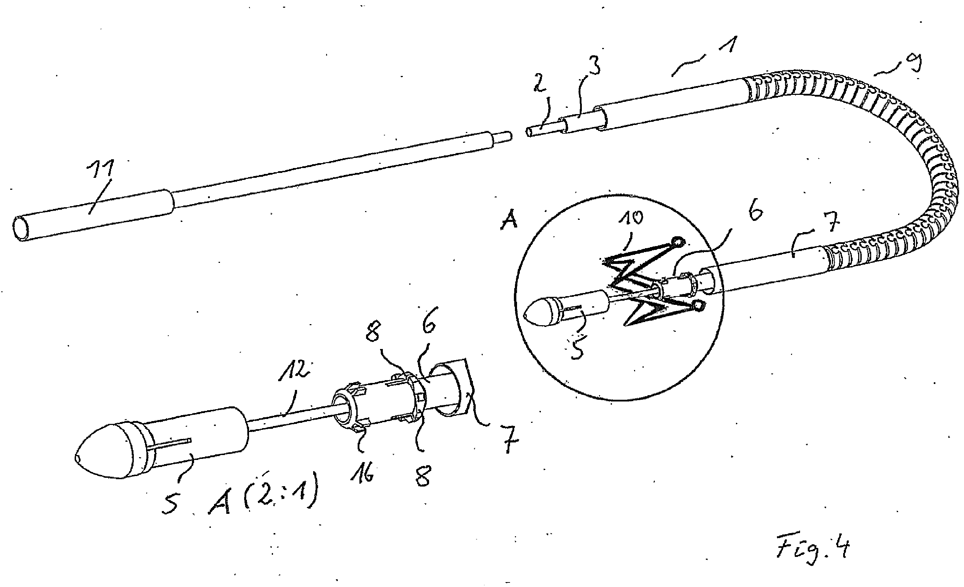

Ergibt sich bei der Funktionsprobe der Herzklappenprothese, dass diese ihre Funktion zumindest in einem ausreichenden Maße erfüllen kann, kann, wie in Figur 4 gezeigt, das Teil/hülsenförmige Element 7 weiter distal zurück und/oder ein weiteres Teil/hülsenförmiges Element 6 der Kartuscheneinheit 4 in proximale Richtung verschoben werden, so dass auch der distale Teil der Verankerungsstütze 10 vollständig frei gegeben und vollständig expandieren kann.

In Figur 4 wird außerdem deutlich, dass an distalen Endbereichen der Verankerungsstütze 10 Ösen oder andere geeignete Elemente ausgebildet worden sind, die vorab in Verankerungselemente 8, die am Teil/hülsenförmigen Element 6 ausgebildet sind, eingegriffen hatten. Über diese Ösen mit den Verankerungselementen 8 kann gesichert werden, dass bei festgestellter Fehl- oder Falschimplantation einer Verankerungsstütze 10 mit Herzklappenprothese ein sicheres Zurück- und Herausziehen, bei Mitführung von Verankerungsstütze 10 und Herzklappenprothese aus dem Körper des Patienten erreichbar ist.

Mittels der Verankerungselemente 8 und gegebenenfalls auch weiteren Führungselementen 16, die am Teil/hülsenförmigen Element 6, der Kartuscheneinheit 4 ausgebildet sein können, besteht auch die Möglichkeit, eine radiale Verdrehung vorzunehmen, um die Bügel einer Verankerungsstütze 10 auch in einer exakten geeigneten Winkellage, beispielsweise in die Taschen einer alten Herzklappenprothese einführen zu können, wobei während der Implantation das gesamte Katheter vom Operateur um seine Längsachse leicht verdreht werden kann.

Insbesondere in der Einzelheit A von Figur 4 ist auch eine Kanüle 12, die durch die Kartuscheneinheit 4 entlang ihrer Längsachse geführt ist, erkennbar. Über die Kanüle 4 kann beispielsweise der im allgemeinen Teil der Beschreibung erwähnte Führungsdraht durch die Kartuscheneinheit 4 geführt sein.

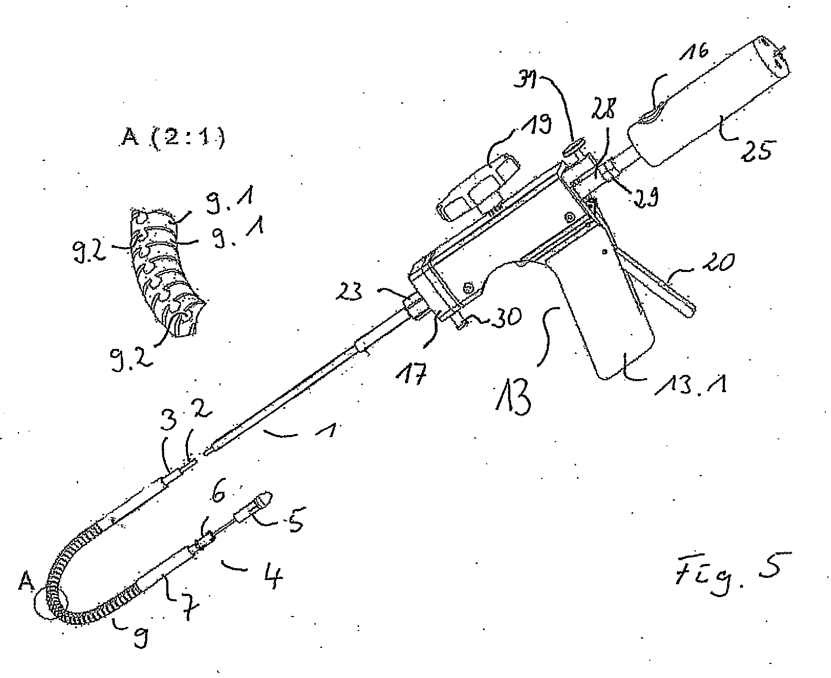

In Figur 5 ist ein Katheters mit Handhabungsteil 13, an dem weitere Bedienungselemente für die Manipulation vorhanden sind, dargestellt.

Dabei entspricht das bereits in den Figuren 1 bis 4 gezeigte Führungssystem 1 mit Kartuscheneinheit 4 auch dem hier gezeigten Beispiel.

Dabei ist mit der Einzelheit A jedoch eine mögliche Ausbildung des biegbaren Bereiches 9, als Gliederkette verdeutlicht worden.

Die einzelnen Glieder 9.1 sind dabei generell jeweils in gleicher Gestalt und Dimensionierung ausgebildet.

Die sich gegenüberliegenden Stirnseiten der einzelnen Glieder 9.1 sind dabei so gestaltet, dass sie Einzelgelenke 9.2 bilden, die jeweils formschlüssig in benachbarte einzelne Glieder 9.1 eingreifen und infolge von Spalten mit jeweils ausreichender Spaltbreite zwischen den einzelnen Gliedern 9.1 eine Biegung des biegbaren Bereiches, um die bereits angesprochenen mindestens 180°, bei einem Radius von ca. 50 mm gewährleisten.

Die Einzelgelenke 9.2 sind dabei durch entsprechende Ausformung der sich jeweils gegenüberliegenden Stirnseiten der einzelnen Glieder 9.1 gebildet worden, wobei eine entsprechende Aussparung an einer Stirnseite und eine entsprechende abgerundete komplementäre ausgebildete Erhebung an der diametral gegenüberliegenden Stirnseite der einzelnen Glieder 9.1 die Einzelgelenke 9.2 an jeweils benachbarten einzelnen Gliedern 9.1 bilden.

Der biegbare Bereich 9 kann in nicht dargestellter Form mit einem Kunststoffschlauch fluiddicht umschlossen sein.

In Figur 5 ist ferner verdeutlicht, wie ein Handhabungsteil 13 für das Einführen und die Manipulation eines erfindungsgemäßen Katheters ausgebildet sein kann.

Für das Einführen und Herausziehen des Katheters mit Führungssystem 1 und Kartuscheneinheit 4 ist ein Handgriff 13.1 vorhanden. Der Handgriff 13.1 des Handhabungsteils 13 dient dabei zum Steuern des eingeführten Katheters.

Im proximalen Teil des Handhabungsteiles 13 ist ein flüssigkeitsdichter Verschluss in Form einer Platte 17 vorhanden, an der die Möglichkeit für eine Anflanschung des Führungssystems 1 mittels einer Überwurfmutter 23 und hier nicht dargestellten Abdichtelementen vorhanden.

Des Weiteren ist ein standardisierter Lueranschluss 30 vorhanden, über den die Kühlflüssigkeit zugeführt werden kann.

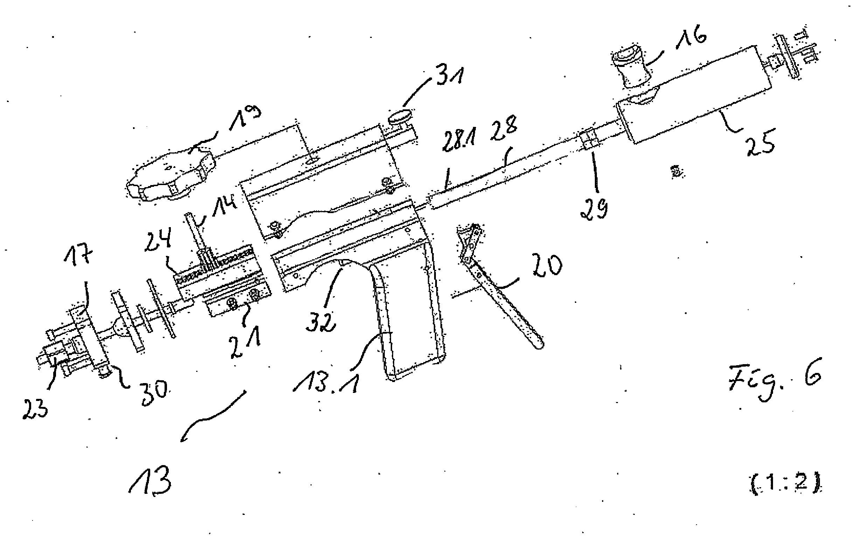

Mit dem Handgriff 19, der um eine Achse verdreht werden kann, kann die jeweilige Krümmung des biegbaren Bereiches 9 über Zugseile (nicht dargestellt) realisiert werden, wobei hierzu nähere Erläuterungen noch bei der Beschreibung der Figur 6 folgen.

Das gesamte Handhabungsteil 13 sollte möglichst flüssigkeits- und gegebenenfalls auch gasdicht gegenüber der Umwelt und dem Führungssystem 1 abgedichtet sein.

Mit dem am Handgriff 13.1 angreifenden Hebels 20 kann eine laterale Bewegung des Rohres 28 in proximaler Richtung erreicht werden, wodurch die entsprechende Bewegung und daraus resultierende Zug- bzw. Druckkraft auf eines der beiden Betätigungselemente 2 und/oder 3 übertragen werden kann, so dass eine Manipulation der einzelnen Teile/hülsenförmigen Elemente 5, 6 und/oder 7 der Kartuscheneinheit 4, wie beispielhaft bereits vorab beschrieben, in fein dosierter Form über pumpenförmige Bewegungen des Hebels 20 erreicht werden können.

Mit dem Drückergriff 25 kann in Verlängerung über den Federstrang, als Betätigungselement 2, die Position des Teiles 5 der Kartuscheneinheit 4 gegenüber dem hülsenförmigen Teil 6 der Kartuscheneinheit 4 mit dem Befestigungshaken, als Verankerungselemente 8 manipuliert werden. Der Drückergriff 25 wird mittels einer Druckfeder in einer gewindeförmigen Verzahnung 28.1 eines Rohres 28 eingerastet. In dieser Weise folgt der Drückergriff 25 der proximalen Bewegung des Rohres 28, das mit dem Teil 6 der Kartusche über den Federstrang, als Betätigungselement 3 verbunden ist.

Nach Erreichen eines Endanschlages zur ersten Entladestufe, kann durch Verdrehung des Drückergriffes 25 eine, im Sinne der Steigung des Gewindes 28.1 fein dosierte axiale Verschiebung des Teiles 5 der Kartuscheneinheit 4 gegenüber dem Teil 6 der Kartuscheneinheit 4 erzielt werden.

Für die Betätigung des Drückergriffes 25 kann dieser das Teil 5 der Kartuscheneinheit 4 hier ohne eine zusätzliche Feinjustierung bewegen.

Mit einer solchen Manipulation kann die Freigabe der Verankerungsstütze 10 (siehe Figur 3) erreicht werden und die Verankerungsstütze 10 ist in dieser Position noch zurückhol bar.

Nach Freigabe des Anschlages 29 durch ein Stellglied 31, dass beispielsweise als Stellschraube ausgeführt ist, kann die Kartuscheneinheit 4 durch Betätigung des Hebelsystems 20, wie vorab beschrieben, weiter ausgefahren werden, bis die Halteösen der Verankerungsstütze 10 die Kartuscheneinheit 4 verlassen haben und so die Verankerungsstütze 10 durch ihre Expansionskräfte von den Verankerungselementen 8 abspringen können.

Die Elemente der Kartuscheneinheit 4 können stufenweise zurückgezogen werden. Dabei kann das Teil 5 der Kartuscheneinheit 4 durch Zurückziehen des Drückergriffes 25 (Drücker eingerastet) bis über das Teil 6 der Kartuscheneinheit 4 zurückgeholt werden.

Durch Betätigung eines Entriegelungsbolzens 32 kann durch weiteres Zurückziehen des Drückergriffes 25 auch das mit dem Rohr 28 verbundene Teil 6 der Kartuscheneinheit 4 in seine Ausgangsposition zurückgeholt werden, so dass dann die Kartuscheneinheit 4 wieder vollständig geschlossen ist. In diesem Zustand kann der Katheter wieder aus dem Körper des Patienten entfernt werden. Mit Figur 6 ist in Form einer Explosionsdarstellung das Handhabungsteil 13 für dieses Beispiel weiter verdeutlicht.

So ist erkennbar, dass durch Drehung des Handrades 19 über die Welle 14 eine Verschiebung von zwei parallel zueinander ausgerichteten Zahnstangen 24 erreicht werden kann. Dabei bewegt sich die eine Zahnstange 24 in proximaler Richtung, wenn sich die parallel dazu ausgerichtete Zahnstange 24 in distale Richtung bewegt.

An entsprechend an den Zahnstangen 24 angreifenden Klemmbacken 21 können hier ebenfalls nicht dargestellte Zugseile befestigt sein, die zum biegbaren Bereich 9 durch das innen hohle Führungssystem 1 geführt und bevorzugt in dessen proximalem Bereich befestigt sind.

So kann durch entsprechende Drehung des Handgriffes 19 auf mindestens eines der beiden Zugseile eine Zugkraft ausgeübt werden, die zur entsprechenden Krümmung des biegbaren Bereiches 9 in dosierter Form führt, so dass das Führungssystem 1 mit der Kartuscheneinheit 4 durch den Aortenbogen in definierter Form geführt werden kann.

In Figur 6 wird weiter deutlich, dass der mit dem Handgriff 13.1 verbundene Hebel 20 über eine feine Verzahnung 28.1 am Rohr 28 angreift und dieses dann entsprechend für die Manipulation, zur sequentiellen Freigabe der Verankerungsstütze 10 über die Betätigungselemente 2 und/oder 3 manipuliert werden kann.

## Patentansprüche

1. System zum Behandeln einer erkrankten Herzklappe, wobei das System folgendes aufweist:
- eine Herzklappenprothese mit einer selbst-expandierbaren Verankerungsstütze; und
- einen Katheter für die transvaskuläre Implantation der Herzklappenprothese mit der selbst-expandierbaren Verankerungsstütze,
wobei der Katheter folgendes aufweist:
a) eine Kartuscheneinheit (4) zur Aufnahme der Verankerungsstütze (10) und Herzklappenprothese in zusammengefalteter Form; und
b) ein innen hohles Führungssystem (1), an dem die Kartuscheneinheit (4) am proximalen Ende lösbar befestigt ist, wobei sich an die Kartuscheneinheit (4) ein biegbarer Bereich (9) anschließt, der so ausgebildet und dimensioniert ist, dass er eine Bewegung des Führungssystems (1) durch den Aortenbogen eines Patienten ermöglicht
**gekennzeichnet durch**
c) ein am distalen Ende des hohlen Führungssystems (1) befestigtes Handhabungsteil (13) mit Bedienelementen (13.1, 20, 25) zum Ausüben von Zug- bzw. Druckkräften auf durch das Führungssystem (1) zur Kartuscheneinheit (4) laufende Betätigungselemente (2, 3) und zum gezielten Manipulieren von Teilen (5, 6, 7) der Kartuscheneinheit (4) derart, dass Einzelteile der Herzklappenprothese mit Verankerungsstütze sequentiell freigebbar sind,
wobei für die sequentielle Freigabe der Einzelteile der Herzklappenprothese mit Verankerungsstütze die Teile (5, 6, 7) der Kartuscheneinheit (4) radial um ihre Längsachse und/oder lateral in proximaler und distaler Richtung bewegt werden,
wobei die Betätigungselemente (2, 3) als Zug- und Druckmittel ausgebildet sind und durch das Innere des Führungssystems (1) geführt sind;
wobei über die Bedienelemente (13.1, 20, 25) eine Bewegung der Betätigungselemente (2, 3) auslösbar ist, wobei die Bewegung der entsprechenden Betätigungselemente (2, 3) in dosierter Form, beispielsweise mit entsprechenden Übersetzungsverhältnissen, erfolgt und zumindest durch Endanschläge (29) oder Rasteinstellungen begrenzt wird.

2. System nach Anspruch 1,
wobei die Verankerungsstütze Stützbügel aufweist, welche ausgebildet sind, um in die Taschen der erkrankten Herzklappe eingeführt zu werden.

3. System nach Anspruch 1 oder 2,
wobei durch das Innere des Katheters ein Führungsdraht geführt ist.

4. System nach einem der vorhergehenden Ansprüche,
wobei am Führungssystem (1) und/oder der Kartuscheneinheit (4) Röntgenstrahlung absorbierende Markierungselemente vorhanden sind.

5. System nach einem der vorhergehenden Ansprüche,
wobei am Katheter ein Schirmfilter vorgesehen ist.

6. System nach einem der vorhergehenden Ansprüche,
wobei ein Ballon im Inneren des Führungssystems (1), der Kartuscheneinheit (4) angeordnet oder durch das Innere des Führungssystems (1) bis hin zur zu expandierenden Verankerungsstütze (10) führbar ist.

7. System nach einem der vorhergehenden Ansprüche,
wobei das Handhabungsteil als Handgriff mit Bedienelementen ausgebildet ist.

8. System nach einem der vorhergehenden Ansprüche,
wobei die Kartuscheneinheit (4) proximalseitig eine Spitze aufweist, welche aus einem flexiblen Werkstoff, beispielsweise Silikon, gebildet ist.

## Claims

1. A system for treating a diseased heart valve, the system comprising:
- a prosthetic heart valve with a self-expandable anchoring support; and
- a catheter for transvascular implantation of the prosthetic heart valve with the self-expandable anchoring support,
wherein the catheter comprises:
a) a cartridge unit (4) for receiving the anchoring support (10) and the prosthetic heart valve in folded form; and
b) an internally hollow guide system (1) to which the cartridge unit (4) is releasably attached at the proximal end, the cartridge unit (4) being adjoined by a bendable portion (9) shaped and dimensioned to allow movement of the guide system (1) through a patient's aortic arch **characterized by**
c) a manipulating part (13) fastened to the distal end of the hollow guide system (1) and having operating elements (13.1, 20, 25) for exerting tensile or compressive forces on actuating elements (2, 3) running through the guide system (1) to the cartridge unit (4) and for selectively manipulating parts (5, 6, 7) of the cartridge unit (4) in such a way that individual parts of the prosthetic heart valve with the anchoring support can be released sequentially,
wherein for the sequential release of the individual parts of the prosthetic heart valve with the anchoring support, the parts (5, 6, 7) of the cartridge unit (4) are moved radially about their longitudinal axis and/or laterally in proximal and distal directions,
wherein the actuating elements (2, 3) are designed as traction and pressure means and are guided through the interior of the guide system (1);
wherein a movement of the actuating elements (2, 3) can be triggered via the operating elements (13.1, 20, 25), wherein the movement of the corresponding actuating elements (2, 3) takes place in a metered form, for example with corresponding transmission ratios, and is limited at least by end stops (29) or detent settings.

2. The system according to claim 1,
wherein the anchoring support comprises support brackets adapted to be inserted into the pockets of the diseased heart valve.

3. The system according to claim 1 or 2,
wherein a guide wire passes through the interior of the catheter.

4. The system according to any of the preceding claims,
wherein X-ray absorbing marking elements are present on the guide system (1) and/or the cartridge unit (4).

5. The system according to any of the preceding claims,
where a screen filter is provided on the catheter.

6. The system according to any of the preceding claims,
wherein a balloon is arranged inside the guide system (1), the cartridge unit (4) or can be guided through the interior of the guide system (1) up to the anchoring support (10) to be expanded.

7. The system according to any of the preceding claims,
wherein the manipulating part is designed as a handle with operating elements.

8. The system according to any of the preceding claims,
wherein the cartridge unit (4) has a proximal tip formed of a flexible material, for example silicone.

## Revendications

1. Système de traitement d'une valvule cardiaque défaillante, dans lequel le système comprend les éléments suivants :
- une prothèse de valvule cardiaque avec un soutien d'ancrage auto-expansible ; et
- un cathéter pour l'implantation transvasculaire de la prothèse de valvule cardiaque avec le soutien d'ancrage auto-expansible,
dans lequel le cathéter comprend les éléments suivants :
a) une unité à cartouche (4) destinée à recevoir le soutien d'ancrage (10) et la prothèse de valvule cardiaque sous forme pliée-groupée ; et
b) un système de guidage creux à l'intérieur (1) au niveau duquel l'unité à cartouche (4) est fixée de façon détachable à l'extrémité proximale, dans lequel une zone flexible (9) est raccordée à l'unité à cartouche (4), qui est réalisée et dimensionnée de manière à permettre un déplacement du système de guidage (1) à travers l'arc aortique d'un patient,
**caractérisé par**
c) une partie de manutention (13) avec des éléments de commande (13.1, 20, 25) fixée à l'extrémité distale du système de guidage creux (1), pour exercer des forces de traction ou de compression sur des éléments de commande (2, 3) qui s'étendent à travers le système de guidage (1) jusqu'à l'unité à cartouche (4), et pour manipuler de façon ciblée des parties (5, 6, 7) de l'unité à cartouche (4), de telle sorte que des parties individuelles de la prothèse de valvule cardiaque avec le soutien d'ancrage peuvent être libérées séquentiellement,
dans lequel, pour la libération séquentielle des parties individuelles de la prothèse de valvule cardiaque avec le soutien d'ancrage, les parties (5, 6, 7) de l'unité à cartouche (4) sont déplacées radialement autour de leur axe longitudinal et/ou latéralement dans une direction proximale et distale, dans lequel les éléments d'actionnement (2, 3) sont réalisés comme moyens de traction et de compression et sont passés à travers l'intérieur du système de guidage (1) ;
dans lequel les éléments de commande (13.1, 20, 25) permettent de déclencher un déplacement des éléments d'actionnement (2, 3), dans lequel le déplacement des éléments d'actionnement (2, 3) correspondants s'effectue sous une forme dosée, par exemple avec des conditions de translation correspondantes, et est limité au moins par des butées terminales (29) ou des positions d'enclenchement.

2. Système selon la revendication 1,
dans lequel le soutien d'ancrage comprend des arceaux d'ancrage qui sont réalisés pour être introduits dans les poches de la valvule cardiaque défaillante.

3. Système selon la revendication 1 ou 2,
dans lequel un fil de guidage est passé à travers l'intérieur du cathéter.

4. Système selon l'une quelconque des revendications précédentes, dans lequel des éléments de marquage qui absorbent les rayons X sont présents sur le système de guidage (1) et/ou sur l'unité à cartouche (4).

5. Système selon l'une quelconque des revendications précédentes, dans lequel un filtre-écran est prévu sur le cathéter.

6. Système selon l'une quelconque des revendications précédentes, dans lequel un ballon est agencé à l'intérieur du système de guidage (1) de l'unité à cartouche (4), ou peut être passé à travers l'intérieur du système de guidage (1) jusqu'au soutien d'ancrage (10) à mettre en expansion.

7. Système selon l'une quelconque des revendications précédentes, dans lequel la partie de manutention est réalisée sous forme de manette avec avec des éléments de commande.

8. Système selon l'une quelconque des revendications précédentes, dans lequel l'unité à cartouche (4) comprend du côté proximal une pointe qui est formée en un matériau flexible, par exemple en silicone.
